(19) Europäisches Patentamt

European Patent Office

Office européen des brevets

(11) EP 1 501 778 B1

(12) EUROPEAN PATENT SPECIFICATION

(45) Date of publication and mention
of the grant of the patent:
**08.03.2006 Bulletin 2006/10**

(51) Int Cl.:
*C07C 51/00* (2006.01)     *C07C 51/265* (2006.01)

(21) Application number: **03731081.0**

(22) Date of filing: **06.05.2003**

(86) International application number:
**PCT/US2003/013772**

(87) International publication number:
**WO 2003/095408 (20.11.2003 Gazette 2003/47)**

(54) **PROCESS FOR THE PRODUCTION OF CARBOXYLIC ACIDS**

VERFAHREN ZUR HERSTELLUNG VON CARBOXYLSÄUREN

PROCEDE DE PRODUCTION D'ACIDES CARBOXYLIQUES

(84) Designated Contracting States:
**AT BE BG CH CY CZ DE DK EE ES FI FR GB GR
HU IE IT LI LU MC NL PT RO SE SI SK TR**

(30) Priority: **06.05.2002 US 378311 P**

(43) Date of publication of application:
**02.02.2005 Bulletin 2005/05**

(73) Proprietor: **E.I. DU PONT DE NEMOURS
AND COMPANY
Wilmington
Delaware 19898 (US)**

(72) Inventors:
• **WHISTON, Keith
Durham DL3 8EY (GB)**
• **JONES, Graham, Howard
Pickering,
North Yorkshire YO18 8HL (GB)**

(74) Representative: **Cockerton, Bruce Roger et al
Carpmaels & Ransford,
43-45 Bloomsbury Square
London WC1A 2RA (GB)**

(56) References cited:
**DE-A- 2 759 025          DE-A- 2 759 026**

## EP 1 501 778 B1

**Description**

<u>CROSS REFERENCE TO RELATED APPLICATION</u>

This application claims priority benefit of U. S. Provisional Patent Application Serial Number 60/378,311 filed 6 May 2002.

<u>FIELD OF THE INVENTION</u>

This invention relates to a process for producing carboxylic acids, particularly terephthalic acid.

<u>DESCRIPTION OF THE PRIOR ART</u>

Terephthalic acid is an important intermediate in the production of polyesters used, for instance, in the manufacture of fibres, bottles and films. Oxidation of para-xylene with molecular oxygen in a lower (e.g. $C_2$-$C_6$) aliphatic monocarboxylic acid, usually acetic acid, as solvent in the presence of a catalyst system containing one or more heavy metals such as cobalt or manganese and a promoter such as bromine is well known as the standard method for the preparation of terephthalic acid. Acetic acid is particularly useful as the solvent since it is relatively resistant to oxidation in comparison with other solvents and increases the activity of the catalytic pathway. Although this method is favoured for the commercial production of terephthalic acid, there remains a problem in that loss of the acetic acid solvent takes place during the reaction. This loss of carbon from the process increases the cost of operation. The acetic acid loss occurs as a result of combustion of acetic acid to form carbon oxides (CO and $CO_2$) and as a result of the formation of methyl acetate and/or methanol as by-product(s). The formation of methyl acetate and/or methanol from acetic acid can account for about 20-30% of the total carbon loss.

The combustion of acetic acid has previously been investigated and various solutions for controlling the combustion have been proposed, for example, specific reaction conditions or a specific catalyst system.

The other principal mechanism for loss of solvent, i.e., the formation of methyl acetate, was addressed in US-4239493 and US-4560793. These documents disclose a process for the production of terephthalic acid in acetic acid solvent, wherein the vapour effluent evolved from the oxidation reaction containing the methylacetate by-product is passed through a condenser which recovers a portion of this by-product as a condensate. The remaining methylacetate in the off-gas from the condenser is recovered by scrubbing the off-gas with acetic acid and the recovered methylacetate is recirculated to the reaction. The resulting increased concentration of methylacetate in the reaction mother liquor has the effect of suppressing the formation, of methylacetate from acetic acid, thereby reducing the amount of acetic acid solvent lost. Roffia P. et al. (Ind. Eng. Chem Res. 1988, 27, 765-770) reports a study on the interdependence of methyl acetate production and process variables and the advantages obtained from recycling methyl acetate to the oxidation reaction.

<u>DESCRIPTION OF THE INVENTION</u>

It is an object of this invention to provide a process for minimising non-productive carbon loss, in particular loss of acetic acid solvent, in the manufacture of a carboxylic acid by catalytic liquid phase oxidation of a corresponding precursor, particularly the manufacture of terephthalic acid by the oxidation of p-xylene.

Accordingly, the present invention provides a process for the production of a carboxylic acid or its ester by catalytic liquid phase oxidation of a corresponding precursor in acetic acid as solvent, said process comprising (i) forming a reaction medium comprising acetic acid, oxidation catalyst, precursor and oxidant; (ii) optionally recycling methyl acetate produced from the acetic acid as a by-product back to the reaction medium; (iii) introducing additional methyl acetate and/or methanol into the reaction medium, said additional methyl acetate and/or methanol being additional to any recovered methyl acetate recycled back to the reaction medium.

The optional recycling of methyl acetate produced as a by-product back to the reaction medium may be achieved by passing the vapour effluent containing the methyl acetate by-product from the reaction medium through a condenser. A portion of said methyl acetate in said vapour is recovered as a condensate from the condenser. At least part and preferably substantially all of the remaining methyl acetate is recovered from the off-gas of the condenser by scrubbing the off-gas with acetic acid. The methyl acetate thereby recovered is re-circulated to the reaction medium.

The methanol or additional methyl acetate may be introduced directly into the reaction medium or may be introduced, for instance, into the acetic acid feed stream prior to or concurrently with entry of the acetic acid feed stream into the reaction medium.

The process of the present invention is advantageous in that it reduces the amount of non-productive carbon loss in the reaction. There is a reduced formation rate of methyl acetate in the oxidation reaction medium and no increase in the formation of carbon oxides (CO and $CO_2$). The process is of beneficial application in two cases.

Firstly in artificially augmenting the concentration of methyl acetate and/or methanol in the reaction medium in an oxidation plant already practising a high degree of methyl acetate recycle, i.e. in a plant practicing a recycle as per the process of US-4329493. An additional benefit in reducing acetic loss is derived in this way.

Secondly, in oxidation plants in which some or all of the methyl acetate by-product is lost from the reaction and fuel value benefit is derived from the increase in methyl acetate in the vapour effluent or vent gas. Thus, this application is suitable for plants where an off-gas abatement facility is employed to which support fuel is routinely added in order to maintain the unit's operating temperature. Such processes are generally used commercially only where the economics of acetic acid and methyl acetate purchase allow it, i.e. where the cost of the support fuel and/or methyl acetate/methanol is low enough to counter balance the acetic acid loss, or for compliance with local environmental regulations to minimise emission of carbon oxides and, for instance, methyl bromide. A plant of this type utilises catalytic combustion, for instance as disclosed in WO-A-96/39595.

The amount of additional methyl acetate and/or methanol is no more than 4 mole %, preferably no more than 3 mole %, and more preferably no more than mole % of the solvent feed. Preferably, the molar concentration is at least 0.5%. Preferably, the molar concentration is 1-2%. If the concentration of added methyl acetate or methanol is too high, detrimental side reactions such as the build-up of formic acid begin to dominate and an increase in the carbon oxides in the vent gas per mole of para-xylene feedstock is observed.

As noted above, the concept of recycling methyl acetate in a terephthalic acid manufacturing process is well-known and practiced commercially and it is in such plants where the present invention is envisaged to be of most benefit. In the known process, methyl acetate is recycled through the oxidation reactor in order to save acetic acid solvent by inhibiting formation of methyl acetate therefrom, as described in US-4239493 and US-4560793 and by Roffia *et al.* In contrast, the present invention introduces additional, non-recycled methyl acetate or methanol into the reactor, and accrues a corresponding additional benefit in reducing acetic loss. Unexpectedly, the process of the present invention achieves this additional benefit without increase in the undesirable formation of carbon oxides as by-products. Thus, there is no increase in the vent concentrations of CO or $CO_2$ per mole of para-xylene feedstock when the concentration of methyl acetate or methanol in the reactor is increased by the introduction of additional methyl acetate or methanol. This result is particularly surprising in view of the prior disclosure by Roffia *et al* who teach that the methanol produced as a result of the methyl acetate recycle is decomposed to CO and $CO_2$. It is therefore unexpected from this prior disclosure that addition of fresh methyl acetate or methanol does not increase the formation of carbon oxides.

In one aspect, the present invention therefore provides an unexpected advantage in decreasing non-productive carbon loss, in particular loss of acetic acid solvent, in a manufacturing process for carboxylic acids which practices methyl acetate recycle. Thus, there is an economic benefit in increasing the methyl acetate or methanol concentration in the feed to the reactor which results in a reduction of methyl acetate formation from acetic acid without increase in CO or $CO_2$ formation per mole of p-xylene feedstock.

In a second aspect, the present invention provides, in plants operating an off-gas abatement facility, economic benefit as a result of the fuel value obtainable from the unexpected absolute increase in volatile organic compounds (VOCs), i.e. methyl acetate and/or methanol, in the vapour effluent or off-gas from the reactor. The absolute increase in VOCs in the vapour effluent or off-gas is derived from the additional methyl acetate or methanol introduced into the system according to the present invention. According to the conventional wisdom, for instance as described by Roffia et al, it would have been expected that any additional methanol and/or methyl acetate would have decomposed to CO and $CO_2$ before reaching the catalytic combustion unit. The present invention teaches that additional methanol and/or methyl acetate introduced into the system is not lost via the formation of carbon oxides but unexpectedly remains available for use as fuel in an off-gas abatement facility, provided that the additional methyl acetate and/or methanol is added in the amounts described herein.

In either aspect, the teaching of the prior art would not have justified this process modification or suggested the resulting economic benefits.

The process of the present invention may also be used in a plant operating both partial methyl acetate recycle and off-gas abatement. Thus, in plants operating at less than 100% recycle there will be some methyl acetate/methanol in the off-gas available for use as fuel in the catalytic combustion unit.

The modification of the manufacturing process described herein is applicable to both new plant designs and retro fitting to existing plants.

The invention is described herein primarily in relation to terephthalic acid. However, it will be appreciated that the following is also applicable to the production of carboxylic acids or their esters generally, particularly phthalic acids or their esters, by catalytic liquid phase oxidation of a corresponding precursor.

In general terms, the catalytic liquid phase oxidation of p-xylene to produce terephathalic acid comprises feeding acetic acid, oxidant, para-xylene and catalyst into an oxidation reactor that is maintained at a temperature in the range from 150°C to 250°C, preferably 175°C to 225°C, and a pressure in the range from 100 to 5000 kPa, preferably 1000 to 3000 kPa. The feed acetic acid : para-xylene ratio is typically less than 5:1.

The oxidation catalyst is preferably a homogeneous catalyst, i.e. it is soluble in the reaction medium comprising solvent and the aromatic carboxylic acid precursor(s). The catalyst typically comprises one or more heavy metal compounds, e.g. cobalt and/or manganese compounds, and may optionally include an oxidation promoter. For instance, the catalyst may take any of the forms that have been used in the liquid phase oxidation of aromatic carboxylic acid precursors in aliphatic carboxylic acid solvent, e.g. bromides, bromoalkanoates or alkanoates (usually $C_1$-$C_4$ alkanoates such as acetates) of cobalt and/or manganese. Compounds of other heavy metals such as vanadium, chromium, iron, molybdenum, a lanthanide such as cerium, zirconium, hafnium, and/or nickel may be used instead of or in addition to cobalt and/or manganese. Advantageously, the catalyst system will include cobalt bromide ($CoBr_2$) and/or manganese bromide ($MnBr_2$). The oxidation promoter where employed may be in the form of elemental bromine, ionic bromide (e.g. HBr, NaBr, KBr, $NH_4Br$) and/or organic bromide (e.g. bromobenzenes, benzyl-bromide, mono- and di-bromoacetic acid, bromoacetyl bromide, tetrabromoethane, ethytene-di-bromide, etc.). Alternatively the oxidation promoter may comprise a ketone, such as methylethyl ketone, or aldehyde, such as acetaldehyde.

The oxidant in the process of the invention is preferably molecular oxygen, e.g. air or oxygen-enriched air. Instead of molecular oxygen, the oxidant may comprise atomic oxygen derived from a compound, e.g. a liquid phase compound at room temperature, containing one or more oxygen atoms per molecule. One such compound is hydrogen peroxide, which acts as a source of oxygen by reaction or decomposition.

Oxidant (preferably molecular oxygen) is added in amounts in excess of the stoichiometric requirements for full conversion of the paraxylene to terephthalic acid, to minimise formation of undesirable by-products, such as color formers. Immediately upon entering the reactor, the paraxylene is thoroughly mixed with the oxygenated solvent to initiate the reaction. The oxidation reaction is exothermic, and heat may be removed by allowing the acetic acid solvent to vaporise. The corresponding vapour is condensed and most of the condensate is refluxed to the reactor, with some condensate being withdrawn to control reactor water concentration (two moles of water are formed per mole of paraxylene reacted). The residence time is typically 30 minutes to 2 hours, depending on the process.

The effluent, i.e. reaction product, from the oxidation reactor is a slurry of crude terephthalic acid (TA) crystals which are recovered from the slurry by filtration, washed, dried and conveyed to storage. They are thereafter fed to a separate purification step or directly to a polymerization process. The main impurity in the crude TA is 4-carboxybenzaldehyde (4-CBA), which is incompletely oxidized paraxylene, although p-tolualdehyde and p-toluic acid can also be present along with undesirable color formers.

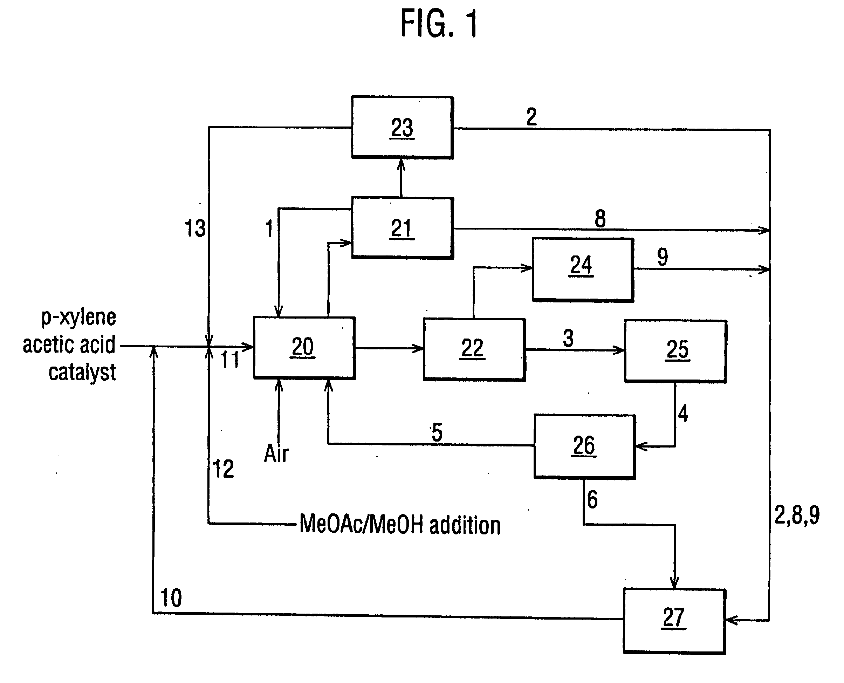

The invention in one of its embodiments is illustrated by Figure 1 showing a p-xylene oxidation process in which full methyl acetate recycle is being practiced. Methyl acetate is conventionally recycled to the oxidation reactor in a number of ways. Methyl acetate in the acetic acid reflux from the primary reactor (20) is directly returned to the reactor (20) from condensor (21) via the reflux return line (1). Vent gasses from the primary reactor and any downstream crystallisation vessels (22) are scrubbed in scrubbers (23 (high pressure) and 24) with acetic acid and water. The extraction solvent from this process is then sent to the solvent recovery process for recovery of acetic acid solvent and methyl acetate (2), or recycled directly to the reactor feed (13).

Methyl acetate present in the product stream (3) from the primary reactor is contained mainly in the process mother liquor. The mother liquor containing methyl acetate (4) is conventionally separated from the product terephthalic acid in filtration unit (25). The filtrate or process mother liquor (4) is then split at stream splitter (26) into two parts: a direct recycle stream (5) and a purge stream (6). The direct recycle stream (5) is then returned, with the contained methyl acetate, directly to the oxidation reactor (20) via the reactor feed. The mother liquor purge stream (6) is sent to a distillation system (27) for the recovery of acetic acid solvent.

The condensate withdrawal (8) normally taken from the overheads condenser system (21) on the primary reactor (20) is also sent to the solvent recovery system (27) together with some or all of the solvent used for scrubbing methyl acetate from the primary reactor vent (2) and from the crystalliser vents (9). A methyl acetate rich stream (10) is then conventionally produced within the solvent recovery system (27) from these combined sources of recovered methyl acetate. This recovered methyl acetate is then recycled to the primary reactor (20) by mixing with directly recycled mother liquor and fresh solvent feed as stream (11). In feeding the oxidation reactor (20), stream (11) can conventionally be combined with streams (5) or (13) and some or all of stream (1) before the primary reactor.

The invention described above constitutes the addition of methyl acetate or methanol (12) into the reactor feed (11) or the mother liquor recycle stream (5) or any combination of these with stream (1), the reflux return.

The invention is further illustrated by the following examples. It will be appreciated that the examples are for illustrative purposes only and are not intended to limit the invention described above. Modification of detail may be made without departing from the scope of the invention.

Experimental

The following examples illustrate how the invention can be demonstrated on a continuous small-scale oxidation facility. It is not practical to recycle methyl acetate directly on a pilot oxidation vessel. This is due to the difficulty of recovering and recycling both the process mother liquor and the volatile components in the vent completely. Therefore it is necessary to simulate methyl acetate recycle on such a unit as described below.

The percentage of methyl acetate (MeOAc) recycle achieved in a process is defined by:

$$\% \text{ Methyl acetate recycle} = (\text{MeOAc in the reactor feed moles per mole}$$
$$\text{p-Xylene/MeOAc leaving the reactor moles per}$$
$$\text{mole p-Xylene}) \times 100$$

In order to simulate methyl acetate recycle on a pilot oxidation unit, one gradually increases the concentration of methyl acetate in the reactor feed by substituting part of the acetic acid solvent with methyl acetate. At the same time the concentration of methyl acetate leaving the unit in the mother liquor, via the condensate withdrawal and through the reactor vents is monitored. As the concentration of methyl acetate in the feed increases, the percentage of simulated recycle also increases until the concentration in the feed exactly balances the measured concentration leaving the oxidation. This state is 100% methyl acetate recycle and simulates the effect of a manufacturing plant recycling all the internally generated methyl acetate until a steady state is reached.

Example 1 below illustrates the invention by showing results from an experiment carried out at above 100% methyl acetate recycle, giving the vent carbon oxides loss and total carbon loss measured for the experiment. Comparative example 1 gives results from an identical experiment where the degree of simulated MeOAc recycle was 89%. Example 1 clearly shows a benefit over comparative example 1 in reducing total carbon loss from the reaction. Example 2 shows results from an identical experiment to example 1 but with no simulated methyl acetate recycle. This further illustrates the benefit of operation at greater than 100% methyl acetate recycle and the advantage of adding some further MeOAc to the reactor solvent feed.

Example 3 and comparative example 2 illustrate that the invention operates using a different catalyst system and under different temperature conditions by comparing an experiment at 101 % simulated methyl acetate recycle with another at 83% methyl acetate recycle as with example 1 and comparative example 1.

Example 4 illustrated how the invention can be practised using methanol (MeOH) rather than MeOAc as the additive.

Results are presented in Tables 1 and 2.

Example 5, comparative example 3 and example 6 show that when higher concentrations of MeOAc and MeOH were included in the reactor feed, catalyst activity deteriorated significantly (as shown by the very significant 4-CBA increase) and the selectivity deteriorated (no coincident reduction in bum was observed).

In comparing results from a p-xylene oxidation reaction for improvements in selectivity or variations in catalyst activity it is important to compare like with like.

Example 1

A zirconium pressure vessel of 5 litre capacity equipped with a stirrer, a reflux condenser with condensate withdrawal facility, an air inlet, a heater, a feed inlet line and a slurry discharge line was charged with 3000 g of an acetic acid solution containing water 8%, methyl acetate 2.3%, cobalt 200 ppm, manganese 400ppm, sodium 100ppm and bromide 800ppm. Cobalt, sodium and manganese were added as their acetate salts. Bromide was added as hydrogen bromide.

The vessel was then heated to 213°C and 19 barg pressure and maintained under these conditions with agitation. An acetic acid feed stream of the following composition was continuously added to the pressure vessel at a rate of 3100 g/hr: p-xylene 18%, water 5.5%, cobalt 120ppm, manganese 240ppm, sodium 60ppm, bromide 490ppm, methyl acetate 2.28% . Air was also added to the vessel at such a rate as to maintain the reactor vent oxygen concentration at 3.5%. Condensate was withdrawn continuously from the pressure vessel at the rate of 1000 g/hr. Product slurry was continuously discharged from the autoclave into a pressure let down vessel before sampling.

After 6 hours of continuous operation, the carbon loss from the reaction was determined. This was achieved by measuring the concentration of by-product carbon oxides, methyl acetate, methanol, methyl bromide, methane and methyl formate in the vent gases discharged from both the oxidation autoclave and let down vessel, and the methyl acetate and methanol in the withdrawn condensate liquid from the reaction and the discharged product slurry. The loss was then calculated as g-atom carbon per mole of p-xylene in the reactor feed. The total concentration of methyl acetate in the reactor feed as g-atom carbon per mole of p-xylene in the feed was then subtracted from this total to give the net carbon loss from the reaction. The percentage of methyl acetate recycle was also calculated from the reactor feed concentration and the measured concentration in the vents, mother liquor and withdrawn condensate samples.

The product terephthalic acid contained 4000 ppm of 4-carboxybenzaldehyde. The extent of simulated methyl acetate recycle was 103%. The total loss of carbon oxides from the reaction was 0.25 moles/mole p-xylene in the feed. The net total carbon loss was 0.25 g-atom carbon/mole p-xylene fed.

Comparative example 1

An experiment as example 1 was carried out but with 1.5% methyl acetate in the reactor charge and 1.5% methyl acetate in the reactor feed. The extent of simulated methyl acetate recycle was 89%. The product terephthalic acid contained 3500ppm of 4-carboxybenzaldehyde. The total loss of carbon oxides from the reaction was 0.26 moles/mole p-xylene in the feed. The net total carbon loss was 0.30 g-atom carbon/mol p-xylene fed.

Example 2

An experiment as example 1 was carried out but with no methyl acetate in either the feed or in the reactor charge. Thus, there was no methyl acetate recycle practiced in this experiment. The product terephthalic acid contained 3500 ppm of 4-carboxybenzaldehyde. The total loss of carbon oxides from the reaction was 0.28 moles/mole p-xylene in the feed. The net total carbon loss was 0.36 g-atom carbon/mol p-xylene fed.

Example 3

An experiment as in example 1 was carried out but at a reactor temperature of 180°C and air was added to the vessel at such a rate as to maintain the reactor vent oxygen concentration at 5%. The initial reactor charge consisted of 3000 g of an acetic solution containing 8% water, 2.5% methyl acetate, 1400ppm cobalt, 470ppm manganese, 100ppm sodium and 1870ppm bromide. A continuous acetic acid feed of the following composition was fed to the autoclave at a rate of 2300 g/hr: p-xylene 18%, water 4.1%, methyl acetate 2%, cobalt 840ppm, manganese 280ppm, sodium 60ppm, bromide 1120ppm. Condensate was withdrawn continuously from the reactor at a rate of 730g/hr.
The terephthalic acid produced had a 4-carboxybenzaldehyde concentration of 3400ppm. The extent of simulated methyl acetate recycle was 101%. The total loss of carbon oxides from the reaction was 0.19 moles/mole p-xylene in the feed. The net total carbon loss was 0.19 g-atom carbon/mol p-xylene fed.

Comparative Example 2

An experiment identical to example 3 was carried out but with 1 % methyl acetate in the autoclave feed during this period. The extent of simulated methyl acetate recycle was 83%. The terephthalic acid produced had a 4-carboxybenzaldehyde concentration of 2800ppm. The total loss of carbon oxides from the reaction was 0.19 moles/mole p-xylene in the feed. The net total carbon loss was 0.22 g-atom carbon/mol p-xylene fed.

Example 4

An experiment as in example 1 was carried out but at a reactor temperature of 185°C and air was added to the vessel at such a rate as to maintain the reactor vent oxygen concentration at 5%. The initial-reactor charge consisted of 3000 g of an acetic solution containing 8% water, 1 % methanol, 900ppm cobalt, 300ppm manganese, 100ppm sodium and 1200ppm bromide. A continuous acetic acid feed of the following composition was fed to the autoclave at a rate of 2300 g/hr; p-xylene 18%, water 4.1 %, methanol 0.85%, cobalt 540ppm, manganese 180ppm, sodium 60ppm, bromide 720 ppm. Condensate was withdrawn continuously from the reactor at a rate of 730g/hr. The methanol addition is the molar equivalent to a concentration of 2% methyl acetate ion the reactor feed. The terephthalic acid produced had a 4-carboxybenzaldehyde concentration of 4300ppm. The total loss of carbon oxides from the reaction was 0.20 moles/mole p-xylene in the feed. The net total carbon loss was 0.09 g-atom carbon/mol p-xylene fed.
Results for the above examples are given in Table 1.

Example 5

An experiment as in example 1 was carried out but air was added to the vessel at such a rate as to maintain the reactor vent oxygen concentration at 1.9%. The initial reactor charge consisted of 3000 g of an acetic solution containing 15% water, 6.3% methyl acetate, 210ppm cobalt, 560ppm manganese, 400ppm sodium and 870ppm bromide. A continuous acetic acid feed of the following composition was fed to the autoclave at a rate of 1850 g/hr : p-xylene 24%, water 4.1 %, methyl acetate 6.3%, cobalt 210ppm, manganese 560ppm, sodium 400ppm, bromide 870ppm. No condensate was withdrawn from the reactor.

The terephthalic acid produced had a 4-carboxybenzaldehyde concentration of 3800ppm. The total loss of carbon oxides from the reaction was 0.39 moles/mole p-xylene in the feed after 6 hours.

Comparative Example 3

An experiment was carried out as in example 5, but no methyl acetate was present in the autoclave charge or reactor feed. The total loss of carbon oxides from the reaction was 0.39 moles/mole p-xylene in the feed after 6 hours, but the terephthalic acid produced had a 4-carboxybenzaldehyde concentration of 2900ppm.

Example 6

An experiment was carried out as in example 5, but 2.74% methanol was present in the autoclave charge and reactor feed instead of the methyl acetate. The total loss of carbon oxides from the reaction was 0.40 moles/mole p-xylene in the feed after 6 hours, but the terephthalic acid produced had a 4-carboxybenzaldehyde concentration of 3900ppm.

The results for examples 5 and 6 and comparative example 3 are given in table 2.

Table 1

| | Product 4CBA | MeOAc in feed | MeOAc in | MeOAc out | $CO+CO_2$ | MeOAc net out | Total carbon by-production | Methyl Acetate Recycle |
|---|---|---|---|---|---|---|---|---|
| | % | ppm | mole/ mole pX | Mole/ mole pX | Mole/ mole pX | mole/ mole pX | g-atom C/ mole pX | % |
| Example 1 | 0.41 | 22830 | 0.193 | 0.187 | 0.254 | -0.006 | 0.246 | 102.0 |
| Comparative Example 1 | 0.36 | 11290 | 0.095 | 0.107 | 0.258 | 0.012 | 0.299 | 89.0 |
| Example 2 | 0.35 | 0 | 0 | 0.026 | 0.275 | 0.026 | 0.356 | 0 |
| Example 3 | 0.28 | 20000 | 0.143 | 0.142 | 0.188 | -0.001 | 0.186 | 101.0 |
| Comparative Example 2 | 0.34 | 10000 | 0.072 | 0.087 | 0.189 | 0.015 | 0.223 | 83.0 |
| Example 4 | 0.43 | 20118 (MeOH) | N/A | 0.102 | 0.205 | N/A | 0.085 | N/A |

Table 2

| | Product 4CBA | MeOAc in feed | $CO+CO_2$ |
|---|---|---|---|
| | % | ppm | mole/mole pX |
| Example 5 | 0.38 | 63300 | 0.39 |
| Comparative Example 5 | 0.28 | 0 | 0.39 |
| Example 6 | 0.38 | 27400 (MeOH) | 0.4 |

**Claims**

1. A process for the production of a carboxylic acid or its ester by catalytic liquid phase oxidation of a corresponding precursor in acetic acid as solvent, said process comprising:

   (i) forming a reaction medium comprising acetic acid, oxidation catalyst, precursor and oxidant;
   (ii) optionally recycling methyl acetate produced from the acetic acid as a by-product back to the reaction medium;
   (iii) introducing additional methyl acetate and/or methanol into the reaction medium, said additional methyl acetate and/or methanol being additional to any recovered methyl acetate recycled back to the reaction medium.

**2.** A process according to claim 1 wherein the carboxylic acid is terephthalic acid and said precursor is p-xylene.

**3.** A process according to claim 1 wherein the methyl acetate produced from the acetic acid as a by-product is recycled back to the reaction medium.

**4.** A process according to claim 1 wherein said recycling of methyl acetate produced as a by-product back to the reaction medium comprises passing the vapour effluent containing the methyl acetate by-product from the reaction medium through a condenser.

**5.** A process according to claim 4 wherein at least a portion of said methyl acetate in said vapour is recovered as a condensate from the condenser.

**6.** A process according to claim 4 wherein at least a portion of the methyl acetate in the off-gas of the condenser is recovered by scrubbing the off-gas with acetic acid and/or water.

**7.** A process according to claim 5 wherein at least a portion of the methyl acetate in the off-gas of the condenser is recovered by scrubbing the off-gas with acetic acid and/or water.

**8.** A process according to claim 6 wherein at least a portion of said methyl acetate and acetic acid resulting from scrubbing the off-gas of the condenser is recycled directly back to the reaction medium.

**9.** A process according to claim 7 wherein at least a portion of said methyl acetate and acetic acid resulting from scrubbing the off-gas of the condenser is recycled directly back to the reaction medium.

**10.** A process according to claim 6 wherein at least a portion of said methyl acetate and acetic acid resulting from scrubbing the off-gas of the condenser is passed to distillation for recovery of acetic acid and methyl acetate and recycle to the reaction medium.

**11.** A process according to claim 7 wherein at least a portion of said methyl acetate and acetic acid resulting from scrubbing the off-gas of the condenser is passed to distillation for recovery of acetic acid and methyl acetate and recycle to the reaction medium.

**12.** A process according to claim 8 wherein at least a portion of said methyl acetate and acetic acid resulting from scrubbing the off-gas of the condenser is passed to distillation for recovery of acetic acid and methyl acetate and recycle to the reaction medium.

**13.** A process according to claim 9 wherein at least a portion of said methyl acetate and acetic acid resulting from scrubbing the off-gas of the condenser is passed to distillation for recovery of acetic acid and methyl acetate and recycle to the reaction medium.

**14.** A process according to claim 1 wherein said process for the production of carboxylic acid comprises crystallising the reaction product in one or more crystallisation vessel(s) and wherein said recycling of methyl acetate comprises scrubbing the off-gas of the crystallisation vessel(s) with acetic acid and/or water.

**15.** A process according to claim 1 wherein said process for the production of carboxylic acid comprises crystallising the reaction product and recovering the carboxylic acid crystals by filtration and wherein said recycling of methyl acetate comprises passing at least a portion of the filtrate to distillation for recovery of acetic acid and methyl acetate and recycle to the reaction medium.

**16.** A process according to claim 15 wherein at least a portion of the filtrate is recycled directly to the reaction medium.

**17.** A process according to claim 1 wherein said additional methyl acetate and/or methanol is no more than 4 mole % of the solvent feed.

**18.** A process according to claim 1 wherein said additional methyl acetate and/or methanol is no more than 3 mole % of the solvent feed.

**19.** A process according to claim 1 wherein said additional methyl acetate and/or methanol is no more than 2 mole % of the solvent feed.

## Revendications

1.  Procédé de production d'un acide carboxylique ou de son ester par oxydation catalytique en phase liquide d'un précurseur correspondant dans l'acide acétique en tant que solvant, ledit procédé comprenant:

    (i) la formation d'un milieu de réaction comprenant l'acide acétique, un catalyseur d'oxydation, un précurseur et un agent oxydant;
    (ii) le recyclage, en option, en retour au milieu de réaction, de l'acétate de méthyle produit à partir de l'acide acétique en tant que produit secondaire;
    (iii) l'introduction d'acétate de méthyle et/ou de méthanol supplémentaire dans le milieu réactionnel, ledit acétate de méthyle et/ou ledit méthanol supplémentaire étant en addition à tout acétate de méthyle récupéré par recyclage en retour au milieu réactionnel.

2.  Procédé selon la revendication 1, dans lequel l'acide carboxylique est l'acide téréphtalique et dans lequel ledit précurseur est le p-xylène.

3.  Procédé selon la revendication 1, dans lequel l'acétate de méthyle produit à partir de l'acide acétique en tant que produit secondaire est recyclé au milieu réactionnel.

4.  Procédé selon la revendication 1, dans lequel ledit recyclage de l'acétate de méthyle produit en tant que produit secondaire en retour au milieu réactionnel comprend le passage, à travers un condenseur, de l'effluent de vapeur contenant le produit secondaire d'acétate de méthyle provenant du milieu réactionnel.

5.  Procédé selon la revendication 4, dans lequel au moins une portion dudit acétate de méthyle dans ladite vapeur est récupérée en tant que condensat en provenance du condenseur.

6.  Procédé selon la revendication 4, dans lequel au moins une portion de l'acétate de méthyle dans le gaz de rejet du condenseur est récupérée par lavage du gaz de rejet à l'aide d'acide acétique et/ou d'eau.

7.  Procédé selon la revendication 5, dans lequel au moins une portion de l'acétate de méthyle dans le gaz de rejet du condenseur est récupérée par lavage du gaz de rejet à l'aide d'acide acétique et/ou d'eau.

8.  Procédé selon la revendication 6, dans lequel au moins une portion dudit acétate de méthyle et dudit acide acétique résultant du lavage du gaz de rejet du condenseur est recyclée directement au milieu réactionnel.

9.  Procédé selon la revendication 7, dans lequel au moins une portion dudit acétate de méthyle et dudit acide acétique résultant du lavage du gaz de rejet du condenseur est recyclée directement au milieu réactionnel.

10. Procédé selon la revendication 6, dans lequel au moins une portion dudit acétate de méthyle et dudit acide acétique résultant du lavage du gaz de rejet du condenseur passe à la distillation en vue de la récupération de l'acide acétique et de l'acétate de méthyle et du recyclage au milieu réactionnel.

11. Procédé selon la revendication 7, dans lequel au moins une portion dudit acétate de méthyle et dudit acide acétique résultant du lavage du gaz de rejet du condenseur passe à la distillation en vue de la récupération de l'acide acétique et de l'acétate de méthyle et du recyclage au milieu réactionnel.

12. Procédé selon la revendication 8, dans lequel au moins une portion dudit acétate de méthyle et dudit acide acétique résultant du lavage du gaz de rejet du condenseur passe à la distillation en vue de la récupération de l'acide acétique et de l'acétate de méthyle et du recyclage au milieu réactionnel.

13. Procédé selon la revendication 9, dans lequel au moins une portion dudit acétate de méthyle et dudit acide acétique résultant du lavage du gaz de rejet du condenseur passe à la distillation en vue de la récupération de l'acide acétique et de l'acétate de méthyle et du recyclage au milieu réactionnel.

14. Procédé selon la revendication 1, dans lequel ledit procédé en vue de la production d'acide carboxylique comprend la cristallisation du produit de réaction dans une ou plusieurs cuves de cristallisation et dans lequel ledit recyclage de l'acétate de méthyle comprend le lavage du gaz de rejet de la ou des cuves de cristallisation à l'aide d'acide acétique ou d'eau.

15. Procédé selon la revendication 1, dans lequel ledit procédé en vue de la production d'acide carboxylique comprend la cristallisation du produit de réaction et la récupération des cristaux d'acide carboxylique par filtration et dans lequel ledit recyclage de l'acétate de méthyle comprend le passage d'au moins une portion du filtrat à la distillation en vue de la récupération de l'acide acétique et de l'acétate de méthyle et du recyclage au milieu réactionnel.

16. Procédé selon la revendication 15, dans lequel au moins une portion du filtrat est recyclé directement au milieu réactionnel.

17. Procédé selon la revendication 1, dans lequel ledit acétate de méthyle et/ou ledit éthanol supplémentaire n'est pas de plus de 4% en moles de l'alimentation en solvant.

18. Procédé selon la revendication 1, dans lequel ledit acétate de méthyle et/ou ledit éthanol supplémentaire n'est pas de plus de 3% en moles de l'alimentation en solvant.

19. Procédé selon la revendication 1, dans lequel ledit acétate de méthyle et/ou ledit éthanol supplémentaire n'est pas de plus de 2% en moles de l'alimentation en solvant.


**Patentansprüche**

1. Verfahren für die Herstellung einer Carbonsäure oder ihres Esters durch katalytische Flüssigphasenoxidation einer entsprechenden Vorstufe in Essigsäure als Lösungsmittel, wobei das Verfahren umfaßt:

   (i) Erzeugen eines Reaktionsmediums, umfassend Essigsäure, Oxidationskatalysator, Vorstufe und Oxidationsmittel;
   (ii) gegebenenfalls Rückführen von Methylacetat, erzeugt aus der Essigsäure als Nebenprodukt, zurück in das Reaktionsmedium;
   (iii) Einführen von zusätzlichem Methylacetat und/oder Methanol in das Reaktionsmedium, wobei das zusätzliche Methylacetat und/oder Methanol zusätzlich zu einem zurückgewonnenen Methylacetat, zurückgeführt in das Reaktionsmedium, ist.

2. Verfahren nach Anspruch 1, wobei die Carbonsäure Terephthalsäure ist und die Vorstufe p-Xylol ist.

3. Verfahren nach Anspruch 1, wobei das Methylacetat, erzeugt aus der Essigsäure als Nebenprodukt, in das Reaktionsmedium zurückgeführt wird.

4. Verfahren nach Anspruch 1, wobei die Rückführung von Methylacetat, erzeugt als Nebenprodukt, zurück in das Reaktionsmedium umfaßt, den Dampfabfluß, enthaltend das Methylacetat-Nebenprodukt, aus dem Reaktionsmedium durch einen Kondensator zu führen.

5. Verfahren nach Anspruch 4, wobei zumindest ein Teil des Methylacetats in dem Dampf als Kondensat aus dem Kondensator zurückgewonnen wird.

6. Verfahren nach Anspruch 4, wobei zumindest ein Teil des Methylacetats in dem Abgas des Kondensators durch Waschen des Abgases mit Essigsäure und/oder Wasser zurückgewonnen wird.

7. Verfahren nach Anspruch 5, wobei zumindest ein Teil des Methylacetats in dem Abgas des Kondensators durch Waschen des Abgases mit Essigsäure und/oder Wasser zurückgewonnen wird.

8. Verfahren nach Anspruch 6, wobei zumindest ein Teil des Methylacetats und der Essigsäure, resultierend aus dem Waschen des Abgases des Kondensators, direkt in das Reaktionsmedium zurückgeführt wird.

9. Verfahren nach Anspruch 7, wobei zumindest ein Teil des Methylacetats und der Essigsäure, resultierend aus dem Waschen des Abgases des Kondensators, direkt in das Reaktionsmedium zurückgeführt wird.

**10.** Verfahren nach Anspruch 6, wobei zumindest ein Teil des Methylacetats und der Essigsäure, resultierend aus dem Waschen des Abgases des Kondensators, zur Rückgewinnung von Essigsäure und Methylacetat und Rückführung in das Reaktionsmedium zur Destillation geführt wird.

**11.** Verfahren nach Anspruch 7, wobei zumindest ein Teil des Methylacetats und der Essigsäure, resultierend aus dem Waschen des Abgases des Kondensators, zur Rückgewinnung von Essigsäure und Methylacetat und Rückführung in das Reaktionsmedium zur Destillation geführt wird.

**12.** Verfahren nach Anspruch 8, wobei zumindest ein Teil des Methylacetats und der Essigsäure, resultierend aus dem Waschen des Abgases des Kondensators, zur Rückgewinnung von Essigsäure und Methylacetat und Rückführung in das Reaktionsmedium zur Destillation geführt wird.

**13.** Verfahren nach Anspruch 9, wobei zumindest ein Teil des Methylacetats und der Essigsäure, resultierend aus dem Waschen des Abgases des Kondensators, zur Rückgewinnung von Essigsäure und Methylacetat und Rückführung in das Reaktionsmedium zur Destillation geführt wird.

**14.** Verfahren nach Anspruch 1, wobei das Verfahren für die Herstellung von Carbonsäure Kristallisieren des Reaktionsprodukts in einem oder mehreren Kristallisationsbehälter(n) umfaßt und wobei die Rückführung von Methylacetat Waschen des Abgases des (der) Kristallisationsbehälter(s) mit Essigsäure und/oder Wasser umfaßt.

**15.** Verfahren nach Anspruch 1, wobei das Verfahren für die Herstellung von Carbonsäure Kristallisieren des Reaktionsprodukts und Gewinnen der Carbonsäurekristalle durch Filtration umfaßt und wobei die Rückführung von Methylacetat umfaßt, zumindest einen Teil des Filtrats zur Rückgewinnung von Essigsäure und Methylacetat und Rückführung in das Reaktionsmedium zur Destillation zu führen.

**16.** Verfahren nach Anspruch 15, wobei zumindest ein Teil des Filtrats direkt in das Reaktionsmedium zurückgeführt wird.

**17.** Verfahren nach Anspruch 1, wobei das zusätzliche Methylacetat und/oder Methanol nicht mehr als 4 Mol-% der Lösungsmittelzufuhr beträgt.

**18.** Verfahren nach Anspruch 1, wobei das zusätzliche Methylacetat und/oder Methanol nicht mehr als 3 Mol-% der Lösungsmittelzufuhr beträgt.

**19.** Verfahren nach Anspruch 1, wobei das zusätzliche Methylacetat und/oder Methanol nicht mehr als 2 Mol-% der Lösungsmittelzufuhr beträgt.

# FIG. 1

EP 1 501 778 B1